# EUROPEAN PATENT SPECIFICATION

(11) **EP 1 026 158 B1**
(45) Date of publication and mention of the grant of the patent: **24.09.2003**
(21) Application number: 99200357.4
(22) Date of filing: 08.02.1999
(51) Int. Cl.: C07D 223/22

(54) **Process for the preparation of 5-carbamoyl-5H-dibenz(b,f)azepine**
Verfahren zur Herstellung von 5-Carbamoyl-5H-dibenz(b,f)azepin
Procédé de préparation de 5-carbamoyl-5H-dibenz(b,f)azépine

(43) Date of publication of application: 09.08.2000
(73) Proprietor: Max India Limited, Nawanshahr, Punjab 144 553 (IN)
(72) Inventor: Vyas, Ketan Dhansukhlal, Kuvempunagar, Mysore 570 023 (IN); Jafri, Wajjid Sajjad, Kuvempu Nagar, Mysore 570 023 (IN); Kulkarni, Ashok Krishna, Kuvempunagar, Mysore 570 023 (IN)
(74) Representative: Davies, Jonathan Mark

(56) References cited:
- EP-A- 0 029 409
- EP-B- 0 277 095
- EP-B- 0 423 679
- EP-B- 0 485 685
- DE-C- 4 307 181
- HAGEMANN H.: 'Kohlensäure-Derivate' HOUBEN-WEYL, METHODEN DER ORGANISCHEN CHEMIE, 4.AUFLAGE vol. E4, pages 343 - 344

## Description

The present invention relates to an improved process for preparing an N-carboxamido-dibenzazepine, particularly carbamazepine, from iminostilbene.

Carbamazepine, or 5-carbamoyl-5H-dibenz(b,f)azepine, is a known muscle relaxant/anticonvulsant eg antiepileptic and psychotropic drug, described in, inter alia, US patent specification no. 2 948 718. The compound is also known as N-carbamoyliminostilbene, and various processes for preparing it from iminostilbene (hereinafter referred to as 'ISB') have been described.

For example, European Patent Specification no. 29 409 discloses the preparation of carbamazepine by reacting ISB with a halocyanogen to produce the 5-cyano derivative, followed by hydrolysation. However, this is an inconvenient 2-stage process that involves the use of toxic reagents. To try to overcome these disadvantages, persons skilled in the art have attempted other methods.

For example, European Patents Specifications nos. 277 095 and 688 768 disclose the reaction of ISB with cyanic acid (HOCN), which may be generated in situ, in an organic solvent in the presence of an acidic agent. An alternative method is disclosed in European Patent Specification no. 423 679, which relates to the chlorocarbonylation and subsequent ammonolysis of ISB; similarly, European Patent Specification no. 485 685 relates to the use of phosgene. These, and other known alternatives, also involve two process steps and/or toxic reagents. surprisingly found that reaction of urea with a protonated form of ISB enables the disadvantages of the prior art to be overcome.

Accordingly, the present invention provides a process for the preparation of carbamazepine, which process comprises reacting, iminostilbene (ISB) or a salt thereof with urea (of formula H₂NCONH₂) or a salt thereof, in a protonating medium, comprising a polar organic solvent and at least a catalytic amount of a proton-donor that can donate a proton to the ISB and/or the urea.

In this way, there is provided a single-step process that can be carried out at moderate temperatures at atmospheric pressure and that requires the use of only 'environmentally-friendly' reagents.

The protonating medium is one that allows protonation of the ISB and/or the urea in the reaction. Preferably, it allows proton transfer from the ISB to the urea. For example, the protonating medium may comprise a polar organic solvent and at least a catalytic amount of proton-donor that can donate a proton to the ISB. Preferably, the proton- donor is an inorganic acid, such as a mineral acid, eg sulphuric, hydrochloric or phosphoric acid. The preferred proton donor is sulphuric acid.

Conveniently, the ISB and proton donor may be present as the corresponding salt of ISB. For example, the process may comprise reaction of a mineral acid salt of ISB, such as ISB.HCl, ISB.H2S04 or ISB.H3PO4, with the urea in a polar organic solvent. Alternatively, the urea and proton donor may be present as the corresponding salt of urea. For example, the process may comprise reaction of a mineral acid salt of urea, such as urea.HCl or urea.H₂S0₄, etc, with the ISB in a polar organic medium. Preferred ISB or urea salts are hydrochlorides.

Preferably, the polar organic solvent is an organic acid, such as an aliphatic carboxylic acid, preferably a C₁₋₄ carboxylic acid, most preferably acetic acid.

Preferably, the molar ratio of ISB:urea used in the reaction is in the range of from 1: 10- 14 (moles), more preferably 1:12-14 (moles).

As well as overcoming the disadvantages of the prior art methods, the process of the present invention provides further advantages in that the preparation of carbamazepine proceeds in near-quantitative yields, and is particularly suitable for large-batch production.

The reaction may be carried out at moderately elevated temperatures (at atmospheric pressure), such as in the range of from 40° to 100°C, preferably in the range of from 80° to 90°C, more preferably from 80-85°C. The reaction may be completed during a period in the range of from 4 to 14 hours, preferably in the range of from 6 to 8 hours, such as from 7 to 8 hours.

Once complete, the reaction mixture may be diluted with water to precipitate the carbamazepine, which may then be separated from the mother liquor by filtration, followed by standard washing and drying procedures.

The present invention further surprisingly provides the use of urea or a salt thereof in the preparation of carbamazepine in the presence of a protonating medium, such as urea (base) in the presence of a proton donor, eg a catalytic amount of a mineral acid.

The carbamazepine, thereby prepared, may then be formulated, for example, by bringing it into association with a suitable carrier therefor, into a pharmaceutical formulation, as is known in the art.

The present invention will now be illustrated by the following non-limiting examples.

### EXAMPLE 1: Preparation of Carbamazepine - Sulphuric Acid Catalyst

To a suspension of urea (400g, 6.66 mols) in acetic acid (500ml), sulphuric acid (15ml) was added, followed by iminostilbene (100g, 0.518 mols), under stirring at 25-30°C. The resulting reaction mixture was heated to 80-85°C and maintained for a period of 7-8 hours, and the reaction was monitored by thin layer chromatography (TLC). The reaction mass was diluted with water, and the resulting carbamazepine product (m.p. 188-189°C) separated by filtration, washed with water until neutral and dried at 90-100°C until constant weight. The identity of the product was further verified by tlc (toluene/methanol 18/03, uv=254nm) to be identical to the reference compound and by infra-red spectroscopy (KBr) Vmax = 3466, 1677, 1605, 1595 cm-1.

### EXAMPLE 2: Preparation of Carbamazepine - Phosphoric Acid Catalyst

To a suspension of urea (80g, 1.333 mols) in acetic acid (100ml), phosphoric acid (8ml) was added, followed by iminostilbene (20g, 0.103 mols), under stirring at 25-30°C. The resulting reaction mixture was worked up according to the method of Example 1 to produce carbamazepine, which was identical to the product of Example 1.

### EXAMPLE 3: Preparation of Carbamazepine using ISB.HCl

To a suspension of urea (80g, 1.333 mols) in acetic acid (100ml), iminostilbene hydrochloride (20.5g, 0.089 mols) was added under stirring at 25-30°C. The resulting reaction mixture was worked up according to the method of Example 1 to produce carbamazepine, which was identical to the product of Example 1.

### EXAMPLE 4: Preparation of Carbamazepine using Urea.HCl

To a suspension of urea hydrochloride (100g/ 1.036 mols) in acetic acid (125ml), iminostilbene (25g, 0.129 mols) was added under stirring at 25-30°C. The resulting reaction mixture was worked up according to the method of Example 1 to produce carbamazepine, which was identical to the product of Example 1.

## Claims

1. A process for the preparation of carbamazepine (5-carbamoyl-5H-dibenz(b,f)azepine), which process comprises reacting iminostilbene (ISB) or a salt thereof with urea (of formula H₂NCONH₂) or a salt thereof, in a protonating medium comprising a polar organic solvent and at least a catalytic amount of a proton donor that can donate a proton to the ISB and/or the urea.

2. A process according to claim 1, wherein the proton donor comprises an inorganic acid.

3. A process according to claim 1 or claim 2, wherein the proton donor comprises sulphuric, hydrochloric or phosphoric acid.

4. A process according to any preceding claim, wherein the ISB and proton donor are present as the corresponding salt of ISB.

5. A process according to any preceding claim, wherein the urea and proton donor are present as the corresponding salt of urea.

6. A process according to any preceding claim, wherein the polar organic solvent is an organic acid.

7. A process according to any preceding claim, wherein the polar organic solvent is acetic acid.

8. A process according to any preceding claim, which comprises the reaction of iminostilbene (ISB) or a salt thereof with urea or a salt thereof in a polar organic solvent, and at least a catalytic amount of a mineral acid.

9. A process according to any preceding claim, wherein the reaction is carried out at a temperature in the range of from 40° to 100°C.

10. A process according to any preceding claim, wherein the molar ratio of ISB : urea used in the reaction is in the range of from 1: 10- 14 (moles).

11. The use of urea or a salt thereof in the preparation of carbamazepine (5-carbamoyl-5H-dibenz(b,f)azepine).

12. A process for the preparation of a pharmaceutical formulation comprising carbamazepine (5-carbamoyl-5H-dibenz(b,f)azepine), which process comprises
(a) preparation of carbamazepine by a process according to any of claims 1 to 10; and
(b) bringing the carbamazepine into association with a pharmaceutically acceptable carrier therefor.

## Patentansprüche

1. Verfahren zur Herstellung von Carbamazepin (5-Carbamoyl-5H-dibenz (b,f) azepin), das das Reagieren von Iminostilben (ISB) oder einem Salz davon mit Harnstoff (der Formel H₂NCONH₂) oder einem Salz davon in einem protonierenden Medium umfasst, das ein polares organisches Lösungsmittel und wenigstens eine katalytische Menge eines Protonenspenders beinhaltet, der ein Proton an das ISB und/oder den Harnstoff abgeben kann.

2. Verfahren nach Anspruch 1, wobei der Protonenspender eine anorganische Säure umfasst.

3. Verfahren nach Anspruch 1 oder Anspruch 2, wobei der Protonenspender Schwefel-, Chlorwasserstoff- oder Phosphorsäure umfasst.

4. Verfahren nach einem der vorherigen Ansprüche, wobei das ISB und der Protonenspender als das entsprechende Salz von ISB vorliegen.

5. Verfahren nach einem der vorherigen Ansprüche, wobei der Harnstoff und Protonenspender als das entsprechende Salz von Harnstoff vorliegen.

6. Verfahren nach einem der vorherigen Ansprüche, wobei das polare organische Lösungsmittel eine organische Säure ist.

7. Verfahren nach einem der vorherigen Ansprüche, wobei das polare organische Lösungsmittel Essigsäure ist.

8. Verfahren nach einem der vorherigen Ansprüche, das das Reagieren von Iminostilben (ISB) oder einem Salz davon mit Harnstoff oder einem Salz davon in einem polaren organischen Lösungsmittel und wenigstens einer katalytischen Menge einer Mineralsäure umfasst.

9. Verfahren nach einem der vorherigen Ansprüche, wobei die Reaktion bei einer Temperatur zwischen 40°C und 100°C stattfindet.

10. Verfahren nach einem der vorherigen Ansprüche, wobei das in der Reaktion verwendete Molverhältnis von ISB zu Harnstoff zwischen 1:10 und 14 (Mol) liegt.

11. Verwendung von Harnstoff oder einem Salz davon zur Herstellung von Carbamazepin (5-Carbamoyl-5Hdibenz(b,f)azepin).

12. Verfahren zur Herstellung einer pharmazeutischen Formulierung, umfassend Carbamazepin (5-Carbamoyl-5Hdibenz(b,f)azepin), umfassend die folgenden Schritte:
(a) Herstellen von Carbamazepin mit einem Verfahren nach einem der Ansprüche 1 bis 10; und
(b) Inkontaktbringen von Carbamazepin mit einem pharmazeutisch akzeptablen Träger dafür.

## Revendications

1. Procédé de préparation de carbamazépine (5-carbamoyl-5H-dibenz(b,f)azépine), lequel procédé comprend mettre à réagir de l'iminostilbène (ISB) ou un sel de celui-ci avec de l'urée (de formule H₂NCONH₂) ou un sel de celle-ci, dans un milieu de protonation comprenant un solvant polaire organique et au moins une quantité catalytique d'un donneur de proton qui peut donner un proton à l'ISB et/ou à l'urée.

2. Procédé selon la revendication 1, dans lequel le donneur de proton comprend un acide inorganique.

3. Procédé selon la revendication 1 ou la revendication 2, dans lequel le donneur de proton comprend de l'acide sulfurique, chlorhydrique ou phosphorique.

4. Procédé selon l'une quelconque des revendications précédentes, dans lequel l'ISB et le donneur de proton sont présents comme le sel correspondant d'ISB.

5. Procédé selon l'une quelconque des revendications précédentes, dans lequel l'urée et le donneur de proton sont présents comme le sel correspondant d'urée.

6. Procédé selon l'une quelconque des revendications précédentes, dans lequel le solvant polaire organique est un acide organique.

7. Procédé selon l'une quelconque des revendications précédentes, dans lequel le solvant polaire organique est de l'acide acétique.

8. Procédé selon l'une quelconque des revendications précédentes, qui comprend la réaction d'iminostilbène (ISB) ou d'un sel de celui-ci, avec de l'urée ou un sel de celle-ci, dans un solvant polaire organique, et au moins une quantité catalytique d'un acide minéral.

9. Procédé selon l'une quelconque des revendications précédentes, dans lequel la réaction est effectuée à une température dans la plage de 40° à 100°C.

10. Procédé selon l'une quelconque des revendications précédentes, dans lequel le rapport molaire d'ISB : urée, utilisé dans la réaction, est dans la plage de 1:10 - 14 (moles).

11. L'utilisation d'urée ou d'un sel de celle-ci dans la préparation de carbamazépine (5-carbamoyl-5H-dibenz(b,f)azépine).

12. Procédé de préparation d'une formulation pharmaceutique comprenant de la carbamazépine (5-carbamoyl-5H-dibenz(b,f)azépine), lequel procédé comprend
(a) la préparation de carbamazépine par un procédé selon l'une quelconque des revendications 1 à 10; et
(b) mettre la carbamazépine en association avec un véhicule pharmaceutiquement acceptable pour ceci.
